Europäisches Patentamt

European Patent Office

Office européen des brevets

(11) Numéro de publication: **0 149 395**
**B1**

(12) **FASCICULE DE BREVET EUROPEEN**

(45) Date de publication du fascicule du brevet:
16.08.89

(51) Int. Cl.⁴: **A 61 K 49/00,** C 12 P 1/04,
A 61 K 39/04

(21) Numéro de dépôt: **84402716.9**

(22) Date de dépôt: **26.12.84**

(54) Procédé de préparation de l'antigène tuberculinique-L.

(30) Priorité: 28.12.83 FR 8320912
25.06.84 BE 900002

(43) Date de publication de la demande:
24.07.85 Bulletin 85/30

(45) Mention de la délivrance du brevet:
16.08.89 Bulletin 89/33

(84) Etats contractants désignés:
AT CH DE FR GB IT LI LU NL SE

(56) Documents cités:
US-A- 4 123 427

C.R. ACAD. SC. PARIS, série III, vol. 293, novembre 1981,
pages 631-634, FR; F. ROMAIN et al.: "Immunologie. -
L'antigène L, nouvel antigène tuberculeux actif en
réaction d'hypersensibilité retardée chez l'animal
sensibilisé par des bacilles vivants (Bacille B.C.G.)"
MICROBIOLOGICAL ABSTRACTS, vol. 6, no. 8, mai
1971, page 33, no. B5595; S. LANDI et al.: "Evaluation of
various substances to prevent adsorption of tuberculin
purified protein derivative (PPD) to glass surfaces" &
BULL. WHO, 43, 91-106, 1970

(73) Titulaire: **INSTITUT PASTEUR, 28, rue du Docteur Roux,
F-75715 Paris Cedex 15 (FR)**

(72) Inventeur: **Augier, Jacques, 207 rue de Vaugirard,
F-75015 Paris (FR)**
Inventeur: **Romain, Félix, 27 rue de Châteaudun,
F-94200 Ivry (FR)**

(74) Mandataire: **Orès, Bernard et al, Cabinet
ORES 6, Avenue de Messine, F-75008 Paris (FR)**

## Description

La présente invention est relative à un nouveau procédé de préparation de l'Antigène tuberculinique L.

La tuberculine est, comme on le sait, un agent de diagnostic permettant de déceler la sensibilisation d'un sujet aux bacilles tuberculeux, qu'il s'agisse de bacilles de Koch virulents ou du vaccin BCG. La sensibilité à la tuberculine se manifeste par un phénomène allergique qui se traduit par une réaction œdémo-érythémateuse consécutive à une application cutanée (par cuti-réaction ou injection intradermique) de tuberculine; cette réaction se manifeste par l'apparition d'un érythème ou d'une papule de forme sensiblement circulaire dont le diamètre est lié à la dose injectée et à la sensibilité du sujet.

Depuis les filtrats concentrés de cultures de bacilles tuberculeux préparés par Koch, on a cherché à purifier la tuberculine de façon de plus en plus poussée, pour en améliorer l'activité et la spécificité soit vis-à-vis de l'espèce bacillaire, et de son état (vivant ou inactivé), soit vis-à-vis de la mesure du degré de résistance du sujet.

Formulant l'hypothèse que la partie active de la tuberculine est constituée par des substances protidiques, les Chercheurs ont, conformément à l'Art antérieur, essentiellement visé à isoler ces substances protidiques sous la forme la plus pure possible, dans le but d'obtenir un produit hautement actif et éviter les réactions non spécifiques de la tuberculine. C'est ainsi que les travaux de Long et Seibert [Cf. Ann. Rev. of Tuberc., 59, 1949, p. 86] ont permis d'isoler un produit de haute activité connu sous le nom de tuberculine PPD (purified protein derivative), qui constitue en outre, un étalon de référence.

Toutefois, comme le procédé d'isolement des protéines proposé par Long et Seibert présentait l'inconvénient de donner des produits présentant des caractères chimiques et une activité biologique variables, J. Bretey et A. Lamensans [Cf. C.R. Acad. Sc, Paris, 16 Mai 1951, p. 1880–1882] proposèrent un autre procédé qui permettait d'extraire des cultures de bacilles tuberculeux, pratiquement la totalité des substances protidiques douées de propriétés tuberculiniques.

Le Brevet américain 4 123 427 fait connaître un procédé de purification d'antigènes protéiques mycobactériens destiné à reconnaître et à mesurer l'hypersensibilité à l'égard des différents bacilles et notamment vis-à-vis de bacilles tuberculeux; ce procédé de préparation d'un antigène pour reconnaître et mesurer l'hypersensibilité lorsqu'on cherche à obtenir une spécificité antigénique utilise la chromatographie d'affinité, à l'aide d'immunoabsorbants et consiste:

– à coupler des anticorps à un substrat inerte, par liaison chimique pour former un absorbant dans lequel l'anticorps spécifique agit comme ligand, puis,

– à préparer des mélanges bruts contenant l'antigène que l'on cherche à obtenir,
– à absorber l'antigène des mélanges bruts qui le contiennent, sur l'absorbant susdit,
– et à éluer l'antigène, puis à le récupérer.

Ce Brevet concerne essentiellement l'obtention d'un immunoabsorbant utilisable pour réaliser la chromatographie d'affinité, en sorte que, s'il a apporté un moyen de purification intéressant, ses limites sont représentées par le fait que cette purification a lieu par immunoabsorption.

Cependant, F. Romain, S. Augier-Gibory, E. Versmisse et J. Augier ont observé récemment [Cf. C.R. Acad. Sc. Paris, 293, 23 Novembre 1981, p. 631–633] que certaines tuberculines présentent une activité 5 à 10 fois plus forte que d'autres, selon la méthode de sensibilisation des animaux utilisés pour le titrage.

L'évaluation de l'activité d'une tuberculine est habituellement réalisée par la mesure du diamètre de la réaction œdémo-érythémateuse provoquée par son injection intradermique chez le cobaye sensibilisé, comparativement à la mesure du diamètre de la réaction obtenue par l'injection d'une tuberculine standard servant de référence. Or, au cours de leurs travaux, F. Romain et Al., ont constaté que certaines tuberculines ont une activité beaucoup plus grande – exprimée par son titre en unités TU/mg – en hypersensibilité retardée chez les cobayes sensibilisés avec des bacilles vivants tels que le bacille BCG, que chez les cobayes sensibilisés par des bacilles inactivés et que l'activité de ces tuberculines peut être 5 à 6 fois supérieure dans le premier cas que ce qu'elle est dans le second cas. Ils ont pu attribuer cette différence d'activité à la présence d'un antigène, dénommé Antigène L, qu'ils ont isolé à partir de tuberculine IP 48 purifiée selon le procédé de J. Bretey et A. Lamensans mentionné plus haut, par chromatographie sur DEAE-Cellulose et traitement de la fraction passant avec le volume mort de la colonne, par concentration, précipitation par l'acétone, dissolution dans l'eau, précipitation par l'acide trichloracétique, séparation du surnageant qui est précipité par l'acétone, puis à nouveau précipité par l'acide trichloracétique, le surnageant obtenu étant précipité par l'acétone pour donner une fraction Aac II contenant l'Antigène L, dont l'activité est 5,57 fois supérieure à celle de la fraction B qui est passée entre 0,005 M et 0,1 M dans la colonne de DEAE-Cellulose et a été concentrée et extraite par entraînement par un précipité d'acide benzoïque. Le titre de la fraction Aac II est de 82 500 TU/mg, et celui de la fraction B est de 14 800 TU/mg; cependant, le rendement en fraction Aac II n'est que de 8% environ.

Ce faible rendement, ajouté à la complexité du procédé, a amené les Inventeurs à rechercher un nouveau procédé d'isolement de l'Antigène L qui permette de réaliser une purification plus poussée encore de la tuberculine de départ et d'obtenir une fraction pratiquement uniquement constituée par de l'Antigène L, présentant un titre plus élevé que précédemment, qui permette d'obtenir des rendements plus élevés en fraction riche en Antigène L et qui soit d'une mise en œuvre relativement simple et rapide.

La présente invention a pour objet un procédé de préparation d'antigène tuberculinique L à partir d'une culture de bacilles tuberculeux, caractérisé en ce qu'on isole une fraction riche en Antigène L, dénommée fraction TP-G-DEAE, dont le titre est supérieur à 100000 U/mg sur des cobayes sensibilisés par du BCG vivant, à partir d'un filtrat de culture de BCG non inactivé soumis à un fractionnement préparatif qui comprend une étape de précipitation dudit filtrat par de l'acide trichloracétique à 2% concentration finale, une étape de précipitation du surnageant recueilli, par de l'acide trichloracétique à 10% concentration finale, reprécipitation du précipité ainsi obtenu, préalablement dissous dans un faible volume d'eau, par de l'acide trichloracétique à 10% concentration finale, pour obtenir une fraction dite TP-G-TCA 10 qui, après dissolution dans un faible volume d'acide acétique et élimination du léger précipité formé, est fractionnée sur une colonne de résine échangeuse d'ions faiblement acide, pour recueillir la fraction correspondant au pic le plus actif sur cobayes sensibilisés par du BCG vivant, passant en fin de volume mort, dénommée fraction TP-G-CM, laquelle fraction est elle-même soumise à un fractionnement sur une colonne de résine échangeuse d'ions basique, pour recueillir la fraction correspondant au pic le plus actif sur cobayes sensibilisés par du BCG vivant, passant en fin de volume mort, dite fraction TP-G-DEAE, dont le titre est supérieur à 100000 U/mg.

Selon un mode de réalisation préféré du procédé objet de la présente invention, les étapes du fractionnement préparatif auquel est soumis le filtrat de culture de BCG non inactivé utilisé comme matière première, pour en isoler la fraction TP-G-DEAE riche en Antigène L, sont réalisées en présence de butanol à 3,8–4,2% concentration finale.

Selon un autre mode de réalisation préféré du procédé objet de la présente invention, la fraction TP-G-DEAE est elle-même soumise à une chromatographie de gel-filtration, par tri moléculaire, pour recueillir la fraction qui passe à la fin du volume mort jusqu'au début de l'inclusion, laquelle fraction est soumise à un dessalage par chromatographie sur tamis moléculaire, pour obtenir une fraction, dénommée TP-G-GF05, dont le titre est supérieur à 600000 U/mg.

Selon un mode de mise en œuvre préféré du procédé objet de la présente invention, la colonne de résine échangeuse d'ions faiblement acide utilisée pour l'obtention de la fraction TP-G-CM, est équilibrée par une solution d'acide acétique 50 mM, pH 3.

Selon un autre mode de mise en œuvre préférée du procédé objet de la présente invention, la colonne de résine échangeuse d'ions basique utilisée pour l'obtention de la fraction TP-G-DEAE, est équilibrée par du tampon TRIS-HCl 50 mM, pH 7.

Les Inventeurs ont pu mettre en évidence que le butanol ajouté à toutes les solutions mises en œuvre dans le procédé d'obtention de la fraction riche en Antigène L, améliore d'une façon déterminante les précipitations, tout en jouant par ailleurs son rôle connu d'antiseptique.

Par unité «U», on entend le 1/40000 de mg de la fraction BR6 décrite sous le nom de «B» dans le C.R. Acad. Sci. 1981, 293, 631–633.

La fraction BR6 a elle-même été titrée en fonction de l'étalon international de PPD humain (PPDM de Copenhague Staten Serum Institute).

Pour la mise en œuvre du procédé conforme à la présente invention, on opère avantageusement comme décrit ci-après:

Preparation de l'antigene L

Le procédé de préparation de l'Antigène L conforme à l'invention, combine un processus de purification par précipitation par l'acide trichloracétique, avec un processus de fractionnement sur colonnes de chromatographie.

1. On part d'un filtrat d'une culture de 12 semaines de BCG non inactivé auquel on ajoute du butanol 4% concentration finale, que l'on précipite par de l'acide trichloracétique à 2% concentration finale; le précipité est écarté et on recueille le surnageant, que l'on précipite par de l'acide trichloracétique à 10% concentration finale. Le précipité obtenu est redissous dans un petit volume d'eau additionnée de butanol 4% concentration finale et à nouveau précipité par de l'acide trichloracétique à 10% concentration finale. Le précipité obtenu est désigné comme étant la fraction «TP-G-TCA 10».

2. On dissout la fraction TP-G-TCA 10 dans une faible quantité d'acide acétique contenant du butanol 4% concentration finale, de préférence 50 mM, pH 3, on élimine par centrifugation le léger précipité qui se forme et on fait passer le surnageant sur une colonne de résine échangeuse d'ions faiblement acide telle que CM-Trisacryl-M («Trisacryl» est une Marque déposée), équilibrée par une solution d'acide acétique contenant du butanol 4% concentration finale, à pH 3, de préférence identique à celle qui a servi à dissoudre la fraction TP-G-TCA 10. Le pic passant en fin de volume mort, désigné comme étant la fraction TP-G-CM, est la fraction la plus active sur cobayes sensibilisés par du BCG vivant.

3. On dissout alors cette fraction TP-G-CM dans une faible quantité de tampon TRIS-HCl contenant du butanol 4% concentration finale 50 mM, pH 7 et on fait passer la solution obtenue sur une colonne de résine échangeuse d'ions basique telle que DEAE-Trisacryl-M (Marque déposée), équilibrée par le même tampon pH 7. Le pic passant en fin de volume mort, désigné comme étant la fraction TP-G-DEAE, est la fraction la plus active sur cobayes sensibilisés par du BCG vivant.

Le procédé combiné d'isolement par précipitation et par fractionnement sur colonne permet d'obtenir la fraction TP-G-DEAE avec un rendement qui est d'au moins 20% par rapport à la fraction TP-G-TCA 10.

4. Le fractionnement est poursuivi en chromatographiant la fraction TP-G-DEAE sur une colonne de tri moléculaire par gel-filtration, telle qu'une colonne d'agarose (type «Sephadex», Marque déposée) ou d'acrylamide-agarose (type «Ultrogel», Marque déposée).

La fraction qui passe à la fin du volume mort jusqu'au début de l'inclusion et qui contient l'antigène L, est recueillie et dessalée par chromatographie sur tamis moléculaire (par exemple sur «Trisacryl» GF05).

La fraction TP-G-GF05 obtenue présente une activité très élevée, très supérieure à celle de la fraction TP-G-DEAE, sur cobayes sensibilisés par du BCG vivant et le rendement en fraction TP-G-GF05 est d'au moins 10% par rapport au TP-G-TCA 10 mis en œuvre en tant que matière première.

On a recherché, parmi les très nombreuses fractions qui avaient été isolées au cours de recherches antérieures sur la purification des PPD, une substance de référence qui soit à peu près indépendante du mode de sensibilisation, que les cobayes soient sensibilisés par des bacilles vivants ou non. Dans ce but, on a comparé pour ces diverses fractions, les diamètres des réactions d'hypersensibilité retardée (HSR) obtenues sur cobayes sensibilisés depuis environ 3 mois, par du BCG vivant ou inactivé. La substance retenue donne, pour une même dose injectée sensiblement (à ±0,5 mm) les mêmes diamètres pour les deux modes de sensibilisation. Il s'agit d'une fraction obtenue par fractionnement sur DEAE-Cellulose, d'une tuberculine humaine purifiée par fractionnement alcoolique d'une solution phénolique de tuberculine IP48, lequel fractionnement donne deux fractions, à savoir:

– une fraction qui traverse la colonne avec le volume mort, dénommée AR6,

– une autre fraction qui est retenue sur la colonne, dont elle est éluable par un tampon phosphate 0,2 M, pH 7, et qui est dénommée BR6.

La fraction AR6 donne des réactions beaucoup plus importantes avec les cobayes sensibilisés par du BCG vivant, tandis que la fraction BR6 donne sensiblement les mêmes diamètres pour les deux modes de sensibilisation (vivant et inactivé). C'est la raison pour laquelle cette fraction BR6 a été choisie comme substance de référence.

L'évaluation de l'activité des fractions isolées, et notamment des fractions TP-G-DEAE et TP-G-GF05, a été mesurée par la détermination du titre de ces fractions par la méthode mise au point par J. Augier, G. Gayot, G. Grance, C. Denise, S. Augier-Gibory et C. Poujeol, décrite dans Develop. Biol. Stand., 29, 1975, page 331.

Les titres des fractions ont été mesurés par rapport au titre de la fraction BR6, respectivement sur des cobayes sensibilisés par du bacille BCG vivant et sur des cobayes sensibilisés par du bacille BCG inactivé. Les résultats obtenus sont réunis dans le Tableau qui va suivre:

Tableau

| Fraction | Titre en HSR avec BCG vivant | Titre en HSR avec BCG inactivé | Rapport Titres vivant/inactivé |
|---|---|---|---|
| Filtrat de culture (de départ) | 15 800 U/ml | 7 100 U/ml | 2,2 |
| TP-G-TCA 10 | 176 000 U/mg | 21 900 U/mg | 8 |
| TP-G-CM | 192 000 U/mg | 11 850 U/mg | 16,2 |
| TP-G-DEAE | 378 000 U/mg | 10 430 U/mg | 36 |
| TP-G-GF05 | 630 000 U/mg | 19 000 U/mg | 33 |

Les fractions TP-G-DEAE et TP-G-GF05 sont celles qui ont le meilleur rapport d'activité, puisqu'elles sont plus de 30 fois plus actives en HSR sur des cobayes sensibilisés par du BCG vivant que sur ceux sensibilisés par du BCG inactivé. Ces fractions TP-G-DEAE et TP-G-GF05 sont donc très riches en Antigène L, la fraction la plus active étant la fraction TP-G-GF05.

L'invention sera mieux comprise à l'aide du complément de description qui va suivre, qui se réfère à des exemples de mise en œuvre du procédé objet de la présente invention.

Il doit être bien entendu, toutefois, que ces exemples de mise en œuvre sont donnés uniquement à titre d'illustration de l'objet de l'invention, dont ils ne constituent en aucune manière une limitation.

Exemple 1

18 litres de milieu de Sauton neutralisés par NH₄OH (pH = 6,9) sont répartis dans 45 boîtes de Roux à raison de 0,4 litre chacune. Ces boîtes de Roux sont ensemencées par des fragments de voile de cultures de BCG de 7 à 8 jours cultivées en ballon. Après 12 semaines de culture à 36–37°C, on mesure le pH de toutes les fioles, et sont retenues celles dont le pH est voisin de 5 ± 0,5. On filtre les bacilles sur papier et on obtient 13 litres de filtrat. Les résidus bacillaires sont éliminés par filtration sur SAM 20. On peut également filtrer sur membrane d'ultra-filtration calibrée à $10^6$ Daltons. Le filtrat est concentré 5 fois par évaporation sous vide et on obtient 2,6 litres auxquels on ajoute du butanol (4% concentration finale). On ajoute ensuite 2% TCA (concentration finale). L'on centrifuge ensuite 1 heure à 15 000 g.

Le surnageant recueilli est refroidi à 4°C, on ajoute du TCA de manière que la concentration finale soit 10% compte-tenu des 2% antérieurs.

Le précipité est recueilli par ultra-filtration et centrifugation, repris dans 15 ml d'H$_2$O distillée, amené à pH 7 par NaOH, clarifié par centrifugation, puis précipité par TCA 10% concentration finale. Le culot est lavé 2 fois par au moins 10 fois le volume du culot, de TCA 10% concentration finale. Il est lavé 3 fois par de l'acétone et est séché sous vide: poids obtenu: 330 mg; il s'agit de la fraction TP-G-TCA 10.

100 mg de ce produit sont dissous dans 4 ml de CH$_3$COOH 50 mM pH 3 contenant éventuellement 4% d'alcool butylique, ou butanol, (concentration finale). On élimine l'insoluble par centrifugation. On dépose le surnageant sur une colonne de CM-Trisacryl-M (Marque déposée) de 12 cm × 1,6 cm, après équilibrage par la même solution de CH$_3$COOH. Le débit est de 5 ml/heure. On recueille le premier pic qui s'élue à la fin du volume mort et le produit est obtenu à l'état sec par lyophilisation (62 mg); il s'agit de la fraction TP-G-CM.

40 mg de ce produit sont dissous dans 3 ml de tampon Tris-HCl 50 mM pH 7 contenant éventuellement 4% (concentration finale) d'alcool butylique (utilisé ici pour ses propriétés antiseptiques) et introduits sur une colonne de DEAE Trisacryl-M de même dimension que la précédente et au même débit; on recueille également le premier pic qui passe avec le volume mort; le produit est lavé 3 fois par ultrafiltration sur membrane UM2 de porosité 1000 Daltons, puis recueilli par lyophilisation. On obtient 21 mg du produit recherché, qui est très riche en Antigène L (fraction TP-G-DEAE). Rendement: 33% par rapport à la fraction TP-G-TCA 10.

Titrage:

Sur cobayes sensibilisés par des bacilles vivants: 378 000 U/mg, et sur cobayes sensibilisés par des bacilles tués et injectés dans de l'antigène de Freund incomplet: 10 430 U/mg. Le rapport vivants/inactivés est de 36.

Analyse chimique:
- teneur en protéines titrées par la méthode du microbiuret: 95%
    - teneur en polyosides libres titrés par la réaction à l'anthrone (Morris): inférieure à 1%
- teneur en ADN titrés par la méthode de Burton: 0
- teneur en ARN titrés par la méthode à l'orcinol: inférieure à 1%

- poids moléculaire mesuré en gel de polyacrylamide en présence de SDS: au maximum 15 000 Daltons
- point isoélectrique mesuré en gel de polyacrylamide en présence de Servalyt 2-11: s'échelonnant de 4 à 6.

Exemple 2

Le processus de fractionnement sur colonnes de chromatographie est poursuivi en soumettant la fraction TP-G-DEAE obtenue à l'Exemple 1 à une chromatographie sur une colonne de tri moléculaire par gel-filtration, par exemple sur une colonne d'«Ultrogel» AcA 202 (Marque déposée-IBF) de 95 cm de hauteur et de 16 mm de diamètre, avec un débit de 3,55 ml/heure/cm².

La fraction qui passe à la fin du volume mort (55 ml) à partir du tube 181 jusqu'au tube 214, c'est-à-dire jusqu'au début de l'inclusion, est recueillie et contient l'antigène L (13,3 ml). La fraction fortement incluse (tubes 215-244; 12,2 ml) est peu active et est éliminée.

La fraction recueillie est dessalée d'une manière connue en elle-même, par chromatographie sur tamis moléculaire, par exemple sur «Trisacryl» GF05, dont le poids d'exclusion est de 3000, sur une colonne de 1 mètre de hauteur et de 16 mm de diamètre.

La fraction TP-G-GF05 obtenue (40 mg) est lyophilisée.

Le rendement en fraction TP-G-GF05 par rapport au TP-G-TCA 10 utilisé comme matière première à l'Exemple 1, est de 15%.

Titrage:
Sur cobayes sensibilisés par des bacilles vivants: 630 000 U/mg
Sur cobayes sensibilisés par des bacilles tués et injectés dans de l'antigène de Freund incomplet: 19 000 U/mg
Rapport vivants/inactivés: 33 U/mg

Caractérisation de la fraction TP-G-GF05 riche en antigène L
- Elle est très soluble, que ce soit dans l'eau, les milieux basiques ou les milieux acides;
- Son poids moléculaire, mesuré sur gel de polyacrylamide à 18%, contenant 0,1% de SDS, est de 15 000 Daltons au maximum;
- Elle est essentiellement constituée par des protéines: 98% par la méthode du biuret;
- Elle ne contient pratiquement pas d'acides aminés aromatiques, comme le montre son spectre d'absorption en U.V.: une très faible absorption à 280 nm;
- Elle ne se fixe pratiquement pas sur les résines échangeuses d'ions faiblement acides et basiques, ce qui permet de la séparer par simple filtration;
- Elle migre par électrophorèse;
- Son point isoélectrique, mesuré sur gel de polyacrylamide en présence de Servalyt 2-11: fait apparaître plusieurs bandes s'échelonnant entre 4 et 6;

- Sa chromatographie HPLC montre qu'elle se compose de plusieurs substances actives.

La fraction TP-G-DEAE présente un titre qui est de très loin supérieur aux titres indiqués dans la littérature pour les fractions actives de la tuberculine et notamment pour les fractions riches en Antigène L, dont le titre est au maximum de 82 500 U/mg, alors que celui de la fraction TP-G-DEAE est supérieur à 100 000 et peut être aussi élevé que 378 000.

La fraction TP-G-GF05 présente, cependant, un titre encore plus élevé que la fraction TP-G-DEAE, puisqu'il peut être environ 1,6 fois supérieur au titre de la fraction TP-G-DEAE, pour un rapport vivants/inactivés sensiblement du même ordre. Ceci permet d'envisager son utilisation en tant qu'agent de diagnostic à des doses très inférieures à celles utilisées actuellement dans le cas de la tuberculine, tout en obtenant une activité et une spécificité très supérieures à celles de la tuberculine.

**Revendications**

1. Procédé de préparation d'antigène tuberculinique L à partir d'une culture de bacilles BCG ou tuberculeux, caractérisé en ce qu'on isole une fraction riche en Antigène L, dénommée fraction TP-G-DEAE, dont le titre est supérieur à 100 000 U/mg, sur des cobayes sensibilisés par du BCG vivant, à partir d'un filtrat de culture de BCG non inactivé soumis à un fractionnement préparatif qui comprend une étape de précipitation dudit filtrat par de l'acide trichloracétique à 2% concentration finale, une étape de précipitation du surnageant recueilli, par de l'acide trichloracétique à 10% concentration finale, reprécipitation du précipité ainsi obtenu, préalablement dissous dans un faible volume d'eau, par de l'acide trichloracétique à 10% concentration finale, pour obtenir une fraction dite TP-G-TCA 10 qui, après dissolution dans un faible volume d'acide acétique et élimination du léger précipité formé, est fractionnée sur une colonne de résine échangeuse d'ions faiblement acide, pour recueillir la fraction correspondant au pic le plus actif sur cobayes sensibilisés par du BCG vivant, passant en fin de volume mort, dénommée fraction TP-G-CM, laquelle fraction est elle-même soumise à un fractionnement sur une colonne de résine échangeuse d'ions basique, pour recueillir la fraction correspondant au pic le plus actif sur cobayes sensibilisés par du BCG vivant, passant en fin de volume mort, dite fraction TP-G-DEAE, dont le titre est supérieur à 100 000 U/mg.

2. Procédé selon la revendication 1, caractérisé en ce que les étapes du fractionnement préparatif auquel est soumis le filtrat de culture de BCG non inactivé utilisé comme matière première, pour en isoler la fraction TP-G-DEAE riche en Antigène L, sont réalisées en présence de butanol à 3,8–4,2% (concentration finale).

3. Procédé selon l'une quelconque des revendications 1 et 2, caractérisé en ce que la fraction TP-G-DEAE obtenue en mettant en œuvre ledit procédé selon la revendication 1, est elle-même soumise à une chromatographie de gelfiltration, par tri moléculaire, pour recueillir la fraction qui passe à la fin du volume mort jusqu'au début de l'inclusion, laquelle fraction est soumise à un dessalage par chromatographie sur tamis moléculaire, pour obtenir une fraction, dénommée TP-G-GF05, dont le titre est supérieur à 600 000 U/mg.

4. Procédé selon l'une quelconque des revendications 1 et 2, caractérisé en ce que la colonne de résine échangeuse d'ions faiblement acide utilisée par l'obtention de la fraction TP-G-CM, est équilibrée par une solution d'acide acétique 50 mM, pH 3.

5. Procédé selon l'une quelconque des revendications 1 et 2, caractérisé en ce que la colonne de résine échangeuse d'ions basique utilisée pour l'obtention de la fraction TP-G-DEAE, est équilibrée par du tampon TRIS-HCl 50 mM, pH 7.

6. Fraction riche en antigène L, caractérisée en ce qu'elle est isolée de filtrat de culture de BCG non inactivé, en mettant en œuvre le procédé selon les revendications 1 à 3 et est constituée par la fraction TP-G-GF05, en ce qu'elle est essentiellement constituée par de l'Antigène tuberculinique L, en ce que son poids moléculaire est de 15 000 Daltons au maximum, en ce que son point isoélectrique est de 4 à 6, en ce qu'elle se compose essentiellement de protéines et présente une faible teneur en acides aminés aromatiques et en ce que son titre d'activité est supérieur à 600 000 U/mg sur des cobayes sensibilisés par du BCG vivant.

7. Agent de diagnostic de la sensibilisation d'un sujet aux bacilles tuberculeux, caractérisé en ce qu'il comprend en tant que constituant actif, la fraction selon la revendication 6.

**Patentansprüche**

1. Verfahren zur Herstellung von Tuberkulin-Antigen L, ausgehend von einer Kultur von BCG- oder Tuberkulose-Bakterien, dadurch gekennzeichnet, dass man eine Fraktion isoliert, die reich ist an Antigen L, bezeichnet Fraktion TP-G-DEAE, deren Titer mehr als 100 000 E/mg beträgt auf mit lebendem BCG sensibilisierten Meerschweinchen, ausgehend von einem Kulturfiltrat von nicht inaktiviertem BCG, unterworfen einer präparativen Fraktionierung, die eine Stufe der Ausfällung des Filtrats mit Trichloressigsäure entsprechend einer Endkonzentration von 2%, eine Stufe der Ausfällung der überstehenden Flüssigkeit mit Trichloressigsäure entsprechend einer Endkonzentration von 10%, Umfällung des so erhaltenen Niederschlags, zuvor gelöst in einem geringen Volumen Wasser, mit Trichloressigsäure entsprechend einer Endkonzentration von 10% umfasst, um eine TP-G-TCA 10 bezeichnete Fraktion zu erhalten, die nach Auflösung in einem geringen Volumen Essigsäure und Abtrennen des entstandenen geringen Niederschlags auf einer Kolonne mit schwach saurem Ionenaustauscherharz fraktioniert wird, um die Fraktion aufzufangen, die dem am stärksten aktiven Pik auf mit lebendem BCG sensibilisierten Meerschweinchen entspricht, am Ende des Todvolumens passiert, Fraktion TP-G-CM bezeichnet, die ihrerseits einer Fraktionierung auf einer Kolonne mit basischem Ionenaustauscherharz unterworfen wird, um die Fraktion aufzufangen, die dem am stärksten aktiven Pik auf mit lebendem BCG sensibilisierten Meerschweinchen entspricht, am Ende des Todvolumens passiert, als Fraktion TP-G-DEAE bezeichnet, deren Titer mehr als 100 000 E/mg beträgt.

2. Verfahren nach Anspruch 1, dadurch gekennzeichnet, dass die Stufen der präparativen Fraktionierung, denen das als Ausgangsmaterial verwendete Kulturfiltrat von nicht inaktiviertem BCG unterworfen wird, um daraus die an Antigen L reiche Fraktion TP-G-DEAE zu isolieren, in Gegenwart von 3,8–4.2% Butanol (Endkonzentration) ausgeführt werden.

3. Verfahren nach einem der Ansprüche 1 und 2, dadurch gekennzeichnet, dass die durch Ausführung des Verfahrens nach Anspruch 1 erhaltene Fraktion TP-G-DEAE ihrerseits einer Gel-Permeations-Chromatographie, mittels Molekülsiebung, unterworfen wird, um die Fraktion aufzufangen, die am Ende des Todvolumens bis zum Beginn des Einschlusses passiert und dass diese Fraktion einer Entsalzung mittels Chromatographie auf Molekularsieb unterworfen wird, um eine TP-G-GF05 bezeichnete Fraktion zu erhalten, deren Titer mehr als 600 000 E/mg beträgt.

4. Verfahren nach einem der Ansprüche 1 und 2, dadurch gekennzeichnet, dass die zur Gewinnung der Fraktion TP-G-CM verwendete Kolonne mit schwach saurem Ionenaustauscherharz mit einer 50 mmolaren Essigsäurelösung, pH 3, äquilibriert wird.

5. Verfahren nach einem der Ansprüche 1 und 2, dadurch gekennzeichnet, dass die zur Gewinnung der Fraktion TP-G-DEAE verwendete Kolonne mit schwach basischem Ionenaustauscherharz mit 50 mmolarem Puffer TRIS-HCl, pH 7, äquilibriert wird.

6. An Antigen L reiche Fraktion, dadurch gekennzeichnet, dass sie isoliert worden ist aus einem Kulturfiltrat von nicht inaktiviertem BCG, gemäss dem Verfahren nach einem der Ansprüche 1 bis 3 und aus der Fraktion TP-G-GF05 besteht, dass sie im wesentlichen aus dem Tuberkulin-Antigen L besteht, dass ihr Molekulargewicht maximal 15 000 Dalton beträgt, dass ihr isoelektrischer Punkt bei 4 bis 6 liegt, dass sie im wesentlichen aus Proteinen zusammengesetzt ist und einen geringen Anteil an aromatischen Aminosäuren aufweist und dass ihr Aktivitätstiter mehr als 600 000 E/mg beträgt, auf mit lebendem BCG sensibilisierten Meerschweinchen.

7. Diagnosemittel für die Sensibilisation eines Lebewesens durch Tuberkulose-Bakterien, dadurch gekennzeichnet, dass es als Wirkstoff die Fraktion nach Anspruch 6 enthält.

## Claims

1. Process for the preparation of tuberculin L antigen from a culture of BCG or tuberculous bacilli, characterized in that a fraction rich in antigen L, called TP-G-DEAE fraction, of which the titer is higher than 100 000 U/mg, in guinea pigs sensitized with living BCG, from an inactivated BCG culture filtrate subjected to a preparatory fractionation which comprises a step of precipitation of said filtrate with trichloracetic acid at 2% final concentration, a step of precipitation of the collected supernatant, with trichloracetic acid at 10% final concentration, reprecipitation of the precipitate so obtained, previously dissolved in a small volume of water, with trichloracetic acid at 10% final concentration, to obtain a fraction called TP-G-TCA 10 which, after dissolving in a small volume of acetic acid and removal of the slight precipitate formed, is fractionated on a weakly acid resin ion exchange column, to collect the fraction corresponding to the peak most active on guinea pigs sensitized with living BCG, passing into the end of the dead space, called fraction TP-G-CM, which fraction itself is subjected to fractionation on a basic resin ion exchange column, to collect the fraction corresponding to the peak most active on guinea pig sensitized with living BCG, passing into the end of the dead space, called fraction TP-G-DEAE, whose titer is higher than 100 000 U/mg.

2. Process according to claim 1, characterized in that the preparatory fractionation steps to which the inactivated BCG culture filtrate is subjected used as starting material, to isolate therefrom the fraction TP-G-DEAE rich in antigen L, are performed in the presence of butanol at 3.8–4.2% (final concentration).

3. Process according to any one of claims 1 and 2, characterized in that the fraction TP-G-DEAE obtained by employing said process according to claim 1, is itself subjected to gel filtration chromatography by molecular sorting, to collect the fraction which passes to the end of the dead space up to the beginning of inclusion, which fraction is subjected to desalting by chromatography on a molecular sieve, to obtain a fraction, called TP-G-GF05, whose titer is higher than 600 000 U/mg.

4. Process according to any one of claims 1 and 2, characterized in that the weakly acid resin ion exchange column used for obtaining the fraction TP-G-CM, is equilibrated with a 50 mM, acetic acid solution, pH 3.

5. Process according to any one of claims 1 and 2, characterized in that the basic resin ion exchange column used for obtaining the fraction TP-G-DEAE, is equilibrated with 50 mM TRIS-HCl buffer, pH 7.

6. Antigen L rich fraction, characterized in that it is isolated from inactivated BCG culture filtrate, by employing the process according to claims 1 to 3 and is constituted by the fraction TP-G-GF05, in that it is essentially constituted by tuberculin L antigen, in that its molecular weight is 15000 Daltons at the maximum, in that its isoelectric point is 4 to 6, in that it is composed essentially of proteins and has a low content of aromatic amino acids and in that its titer of activity is higher than 600000 U/mg in guinea pigs sensitized with living BCG.

7. A diagnostic agent of the sensitization of a subject to tuberculous bacilli, characterized in that it comprises as active constituent, the fraction according to claim 6.